# EUROPÄISCHE PATENTSCHRIFT

(11) **EP 1 015 404 B1**
(45) Veröffentlichungstag und Bekanntmachung des Hinweises auf die Patenterteilung: **14.07.2004**
(21) Anmeldenummer: 98945279.2
(22) Anmeldetag: 24.08.1998
(51) Int. Cl.: C07C 43/11, B01F 17/00

(54) **KÄLTESTABILE FETTALKOHOLALKOXYLATE**
COLD-STABLE FATTY ALCOHOL ALKOXYLATES
ALCOXYLATS D'ALCOOLS GRAS STABLES AU FROID

(30) Priorität: 01.09.1997 DE 19738108
(43) Veröffentlichungstag der Anmeldung: 05.07.2000
(73) Patentinhaber: Cognis Deutschland GmbH & Co. KG, 40589 Düsseldorf (DE)
(72) Erfinder: BEHLER, Ansgar, D-46240 Bottrop (DE); SCHARES, Horst-Dieter, D-40699 Erkrath (DE)
(86) Internationale Anmeldenummer: PCT/EP1998/005355
(87) Internationale Veröffentlichungsnummer: WO 1999/011593

(56) Entgegenhaltungen:
- EP-A- 0 019 173
- EP-A- 0 086 493
- GB-A- 1 172 931
- US-A- 3 382 285
- US-A- 3 770 701
- US-A- 4 093 418
- PATENT ABSTRACTS OF JAPAN vol. 96, no. 3, 29. März 1996 & JP 07 303825 A (LION CORP), 21. November 1995 in der Anmeldung erwähnt

## Beschreibung

Die vorliegende Anmeldung betrifft kältestabile Randompolymerisate von Fettalkoholen mit Ethylenoxid und Propylenoxid, wobei Ethylenoxid und Propylenoxid in einem ganz bestimmten ausgewählten Mengenverhältnis vorliegt, ein Verfahren zur Herstellung derartiger Verbindungen sowie die Verwendung derartiger Verbindungen als oberflächenaktive Mittel in mit Wasser verdünnbaren Wirkstoffkonzentraten, insbesondere von Pestiziden und Agrarchemilkalien.

Alkoholalkoxylate, die auch als Alkylpolyglykolether bezeichnet werden, sind seit Jahrzehnten bekannte Verbindungen, die durch Umsetzung von Alkoholen mit Ethylenoxid und/oder Propylenoxid gewonnen werden. Die Umsetzung erfolgt bei erhöhten Temperaturen und Drucken in Anwesenheit saurer oder alkalischer Katalysatoren. Besondere praktische Bedeutung hat die Verwendung von basischen Verbindungen der Alkali- und Erdalkalimetalle für die Alkoxylierung von Fettalkoholen. Geeignete basische Verbindungen sind Alkali- und Erdalkalialkoxylate wie Natriummethylat und Kaliummethylat oder Alkali- und Erdalkalihydroxide wie Natriumhydroxid und Kaliumhydroxid. In letzter Zeit wird der Verwendung der Alkali- und Erdalkalihydroxide als basische Katalysatoren den Vorzug gegeben, da bei Einsatz von Alkali- und Erdalkalialkoxylaten organische Lösemitteln wie Methanol zwingend sind. Bei den Alkali- und Erdalkalihydroxiden dagegen läuft die Umsetzung in guten Ausbeuten auch im wäßrigen Medium ab.

Bei dem Einsatz wäßriger Lösungen von Alkali- und Erdalkalihydroxiden als basische Katalysatoren werden jedoch insbesondere bei der Ethoxylierung der Fettalkohole Produkte erhalten, die ein ungünstiges Kälteverhalten zeigen und oft schon bei Raumtemperatur zu AusfaHungen neigen. Dies ist unter anderem darauf zurückwführen, daß die Anwesenheit wäßriger Lösungen von Alkali- und Erdalkalihydroxiden die Bildung von hochmolekularen Polyethylenglykolen als Nebenprodukte begünstigt. Diese Polyethylenglykole lassen sich zwar prinzipiell durch Extraktion mit geeigneten Lösemitteln wie Wasser entfernen, aber hierzu bedarf es einen weiteren Prozeßschritt, der sehr zeitaufwendig und zudem nicht universell anwendbar ist.

Gemäß dem Abstract zur japanischen Anmeldung 07,303,825 aus der Zeitschrift CA Selects in : Alkoxylated Oleochemicals, Issue 1996, Seite 5, Hrsg. von der American Chemical Society, Columbus Ohio, Nr. 124, zeigen Randomaddukte von Alkoholen mit 8 bis 18 Kohlenstoffatomen, die 5 bis 15 Mol Ethylenoxid und 0,3 bis 5,0 Mol Propylenoxid enthalten, ein verbessertes Fließverhalten bei niedrigen Temperaturen. In dem einzigen angeführten Beispiel wird Laurylalkohol mit etwa 9 Mol Ethylenoxid und etwa 2,4 Mol Propylenoxid in Anwesenheit von Kaliumhydroxid als basischen Katalysator umgesetzt, wobei ein Produkt erhalten wird, welches einen Fießpunkt von 7,5°C aufweist. Bei kühler Lagerung unter 0°C kommt es aber auch bei diesem Produkt zu Ausfällungen, die störend sind.

Aus dem Dokument US 4,093,418 wird im Beispiel 1 ein Randompolymerisat offenbart, welches insgesamt 57,6 Gew.-% Ethylen- und Propylenoxideeinheiten enthält. Aus der GB-A-1,172,931 sind ethoxylierte und propoxylierte Randompolymerisate bekannt die als oberflächenaktive Mittel in einem Reinigungsmittel verwendet werden. Die EP 0 086 493 offenbart ethoxylierte und propoxylierte oberflächenaktive nichtionische Tenside für den Einsatz in automatischen Geschirrspülmitteln.

Die Aufgabe der vorliegenden Erfindung bestand darin, Fettalkoholalkoxylate zur Verfügung zu stellen, die ein sehr günstiges Kälteverhalten zeigen und nicht zu Ausfällungen neigen. Das Kälteverhalten sollte derart ausgeprägt sein, daß die Produkte selbst bei Temperaturen unter 0°C den Zustand der Klarflüssigkeit zeigen, damit auch bei kühler Lagerung der Produkte keine Ausfällungen auftreten. Weiterhin war gewünscht, daß die erhaltenen Produkte eine sehr gute Auflösbarkeit in kaltem Wasser zeigen, damit sie einsetzbar sind als. oberflächenaktive Verbindungen Zudem sollten die Verbindungen in Anwesenheit wäßriger Lösungen von Basen hergestellt worden sein.

Die, Aufgabe konnte überraschenderweise durch Randompolymerisaten von Fettalkoholen gelöst werden, die in einem bestimmten ausgewählten Verhältnis Ethylenoxid und Propylenoxid randompolymerisiert enthalten.

Ein Gegenstand der vorliegenden Erfindung betrifft daher die Verwendung in Randompolymerisate von Fettalkoholen mit Ethylenoxid und Propylenoxid der Formel (I)

R¹O(EO)ₙ(PO)ₘH (I)

in der
R¹ für einen Alkylrest mit 6 bis 22 Kohlenstoffatomen
EO für CH₂CH₂O
PO für CHCH₃CH₂O und/oder CH₂CHCH₃O
n für eine ganze oder gebrochene Zahl von 2 bis 7 und
m für eine ganze oder gebrochene Zahl von 1,5 bis 3 steht,
wobei das Molverhältnis von Propylenoxid zu Ethylenoxid im Bereich von 10:90 bis 50: 50 liegt, als oberflächenaktives Mittel in mit Wasser verdünnbaren Wirkstoffkonzentraten, dadurch gekennzeichnet, daß die Wirkstoffkonzentrate Pestizide oder Agrarchemikalien enthalten. Besonders bevorzugt werden Randompolymerisate von Fettalkoholen der Formel (I), bei denen das Molverhältnis von Propylenoxid zu Ethylenoxid im Bereich von 25 : 75 bis 40 : 60 liegt.

Des weiteren werden solche Randompolymerisate von Fettalkoholen der Formel (I) bevorzugt, in der in Formel (I) n eine ganze oder gebrochene Zahl von 3 bis 5 ist, sowie Randompolymerisate der Formel (I), in der m eine ganze oder gebrochene Zahl von 2 bis 2,5 ist.

R¹ in Formel (I) leitet sich von Fettalkoholen der Formel R¹OH ab, die 6 bis 22 Kohlenstoffatome aufweisen. Unter Fettalkohole sind primäre aliphatische Alkohole zu verstehen, in der R¹ für einen aliphatischen, linearen oder verzweigten Kohlenwasserstoffrest mit 6 bis 22 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylaikohol, Elaeostearylalkohol, Arachylalkohol, Gadoleylalkohol, Behenylalkohol, Erucylalkohol und Brassidylalkohol sowie deren technische Mischungen, die z.B. bei der Hoehdruckhydrierung von technischen Methylestem auf Basis von Fetten und Ölen oder Aldehyden aus der Roelen'schen Oxosynthese sowie als Monomerfraklion bei der Dimerisierung von ungesättigten Fettalkoholen anfallen.

Bevorzugt im Sinne der Erfindung sind technische Fettalkohole, die zu mindestens 30 Gew.% Fettalkohole mit 14 bis 18 Kohlenstoffatomen und zu höchstens 70 Gew.% Fettalkohole mit 6 bis 12 Kohlenstoffatomen enthalten. Die Gewichtsangaben beziehen sich dabei auf die Fettalkoholmischung. Beispiele für derartige Fettalkoholmischungen sind Kokos-, Palm-, Palmkern- oder Talgfettalkohol.

Die erfindungsgemäßen Randompolymerisate zeichnen sich durch ein sehr gutes Kälteverhalten aus, d.h. selbst bei Temperaturen unter 0°C liegen fließfähige, bevorzugt klare Produkte vor. Die Bestimmung des Kälteverhaltens erfolgt dabei durch Bestimmung des Kältetrübungspunktes gemäß DIN ISO 3015. Weiterhin zeichnen sich die erfindungsgemäßen Produkte durch ein sehr gutes Löseverhalten in kaltem Wasser aus, d.h. sie besitzen sehr niedrige Außösezeiten. Bestimmt wurde das Löseverhalten durch Messung der Zeit, bis sich 10 g der Verbindung in 90 g entionisiertem Wasser (Temperatur: 23 °C) unter Rühren optisch klar auflösten.

Die Randompolymerisation kann durch ein an sich bekanntes Verfahren dargestelt werden, bei den man die bereits beschriebenen Alkohole der Formel R'OH in Gegenwart wäßriger Basen in einem Druckgefäß bei Temperaturen im Bereich von 120 bis 190 °C und einem Druck von 3 bis 5 bar mit Propylen- und Ethylenoxid umsetzt. Als basische Verbindungen sind im Sinne der Erfindung wäßrige Lösungen von Alkali- und/oder Erdalkalihydroxiden einzusetzen, beispielsweise Kaliumhydroxid. Die Hydroxide werden üblicherweise in Mengen von 0,2 bis 5 Gew.%, vorzugsweise 0,3 bis 1,5 Gew.% - berechnet als Hydroxid und bezogen auf Gesamtansatz - eingesetzt. Es empfiehlt sich, die Hydroxide in Form 40 bis 60 gew.%iger-wäßriger-Lösungen einzusetzen. Propylen- und Ethylenoxid werden zusammen mit den Fettalkoholen zur Reaktion gebracht. Dabei können sie separat über zwei verschiedene Düsen in den Reaktionsbehälter eingespeist werden oder aber auch vorher in einem Mischungsbehältnis vorgemischt und anschließend eingespeist werden. Erfindungswesentlich ist dabei, daß Propylenoxid und Ethylenoxid in den oben angegebenen Mengen und angegebenen Mischungsverhältnissen eingesetzt werden. In der Regel werden 2 bis 7 Mol Ethylenoxid , vorzugsweise 3 bis 5 Mol Ethylenoxid pro Mol Fettalkohol eingesetzt sowie 1,5 bis 3 Mol Propylenoxid, vorzugsweise 2 bis 2, 5 Mol, pro Mol Fettalkohol. Besondere Bedeutung hat das molare Mischungsverhältnis von Propylenoxid zu Ethylenoxid, das vorzugsweise im Bereich von 25 : 75 bis 40 : 60 liegt. Durch die gemeinsame Umsetzung von Ethylenoxid und Propylenoxid mit den Fettalkoholen werden sogenannte Randompolymerisate erhalten, d.h. die Anlagerung erfolgt in statistischer Verteilung.

Falls gewünscht, kann sich dem Verfahren eine Neutralisation des basischen Katalysators anschließen. Die Neutralisation kann mit anorganischen und/oder organischen Säuren wie Milchsäure, Oxalsäure, Citronensäure, Essigsäure, Phosphorsäure oder Methansulfonsäure erfolgen. Eine Neutralisation auf Werte von etwa 6,5 bis 7,5 empfiehlt sich in der Regel dann, wenn ungesättigte Fettalkohole eingesetzt werden, da sich diese leicht unter Einwirkung von Luftsauerstoff verfärben. Die Produkte fallen als 100 %ige klare Flüssigkeit an.

Die vorliegenden Erfindung betrifft die Verwendung der oben beschriebenen Randompolymerisaten von Fettalkoholen als oberflächenaktive Mittel in mit Wasser verdünnbaren Wirkstoffkonzentraten, in Konzentraten von Pestiziden und Agrarchemikalien. In jüngster Zeit werden beispielsweise Wasch- und Reinigungsmittel verstärkt in konzentrierter Form angeboten, die nur wenig Wasser enthalten. Derartige Konzentrate können vom Endanwender vor Gebrauch mit Wasser verdünnt werden. Es wird dabei erwartet, daß sich die Konzentrate schnell und ohne Bildung von Ausfällungen auflösen. Durch Verwendung der erfindungsgemäßen Verbindungen als oberflächenaktives Mittel wird dies erreicht.

In Pestizid- und Agrarchemikalienkonzentraten können die erfindungsgemäßen Produkte in Mengen von 0,1 bis 15 Gew.% - bezogen auf Wirkstoff im Konzentrat - verwendet werden. Auch hier können in den Konzentraten übliche Bestandteile in üblichen Mengen enthalten sein.

### Beispiele

### 1. Herstellung eines Randompolymerisates eines C12/14-Alkohol+5EO+2EO

366,3 g (1, 89 Mol) eines C12/C14- Fettalkoholgemisches (etwa 40 Gew.% C12 und 60 Gew.% C14) wurden mit 5 einer 50 Gew.%igen wäßrigen Kaliumhydroxid-Lösung in einem Druckbehälter vorgelegt. Der Behälter wurde 30 Minuten bei 100 °C evakuiert und anschließend mit Stickstoff belüftet. Es erfolgte bei 120 °C eine Zudosierung einer Mischung von 414,9 g (9,43 Mol) Ethylenoxid und 218,8 g (3,77 Mol) Propylenoxid. Der Druck betrug maximal 5 bar. Nach Beendigung der Umsetzung wurde noch eine Stunde bei 120 °C nachreagiert und nochmals 30 Minuten bei 120°C die Apparatur evakuiert. Das erhaltene Produkt wurde mit Milchsäure neutralisiert.

Man erhielt ein klares flüssiges Produkt mit einem Kältetrübungspunkt von -4°C; die Auflösezeit in Wasser betrug 5 Sekunden.

### 2. Herstellung eines Randompolymerisates eines C12/14-Alkohol+3EO+2PO

Analog Beispiel 1 wurden 439,1 g (2,26 Mol) eines C12/C14-Fettalkohols mit einer Mischung von 298,5 g (6,78 Mol) Ethylenoxid und 262,4 g (4.52 Mol) Propylenoxid in Gegenwart von 5 g einer 50 gew.%igen wäßrigen Kaliumhydroxid-Lösung umgesetzt.

Man erhielt ein klares flüssiges Produkt mit einem Kältetrübungspunkt von -14 °C und die Auflösezeit in Wasser betrug 3 Sekunden.

### Vergleichsbeispiel 1 Blockpolymerisat eines C12/14-Alkohols mit 1PO+5EO+1PO

In Anlehnung an Beispiel 1 wurde die in Beispiel 1 beschriebene Mengen an Fettalkohol zunächst bei 120°C mit 109,4 g (1,89 Mol) Propylenoxid, dann bei 180°C mit 414,9 g (9,43 Mol) Ethylenoxid und nach vollständiger Abreaktion erneut bei 120 °C mit 109,4 g (1,89 Mol) Propylenoxid umgesetzt.

Man erhielt ein klares flüssiges Produkt mit einem Kältetrübungspunkt von 1.5 °C und die Auflösezeit in Wasser betrug 75 Sekunden.

### Vergleichsbeispiel 2 Blockpolymerisat eines C12/14- Alkohols mit 5EO+2PO

In Anlehnung an Beispiel 1 wurde die in Beispiel 1 beschriebene Menge an Fettalkohol zunächst bei 180°C mit 414,9 g (9,43 Mol) Ethylenoxid und anschließend bei 120 °C mit 218,8 g (3,72 Mol) Propylenoxid umgesetzt.

Man erhielt ein klares flüssiges Produkt mit einem Kältetrübungspunkt von 7,5 °C und die Auflösezeit in Wasser betrug 8 Sekunden.

## Patentansprüche

1. Verwendung von Randompolymerisaten von Fettalkoholen mit Ethylenoxid und Propylenoxid der Formel (1),
R¹O(EO)ₙ(PO)ₘH (I)
in der R¹ für einen Alkylrest mit 6 bis 22 Kohlenstoffatomen
EO für CH₂CH₂O
PO für CHCH₃CH₂O und/oder CH₂CHCH₃O
n für eine ganze oder gebrochene Zahl von 2 bis 7 und
m für eine ganze oder gebrochene Zahl von 1,5 bis 3 steht,
und das Molverhältnis von Propylenoxid zu Ethylenoxid im Bereich von 10: 90 bis 50 : 50 liegt, als oberflächenaktives Mittel in mit Wasser verdünnbaren Wirkstoffkonzentraten, **dadurch gekennzeichnet, dass** die Wirkstoffkonzentrate Pestizide oder Agrarchemikalien enthalten.

2. Verwendung nach Anspruch 1, **dadurch gekennzeichnet, dass** das Molverhältnis von Propylenoxid zu Ethylenoxid im Bereich von 25 : 75 bis 40 : 60 liegt.

3. Verwendung nach Anspruch 1, **dadurch gekennzeichnet, dass** n eine ganze oder gebrochene Zahl im Bereich von 3 bis 5 ist.

4. Verwendung nach Anspruch 1, **dadurch gekennzeichnet, dass** m eine ganze oder gebrochene Zahl im Bereich von 2 bis 2,5 ist.

5. Verwendung nach Anspruch 1, **dadurch gekennzeichnet, dass** R¹ abgeleitet ist von einer Fettalkoholmischung, die mindestens zu 30 Gew.-% Fettalkohole mit 14 bis 18 Kohlenstoffatomen und höchstens 70 Gew.-% Fettalkohole mit 6 bis 12 Kohlenstoffatomen enthält.

## Claims

1. The use of random polymers of fatty alcohols with ethylene oxide and propylene oxide corresponding to formula (I):
R¹O(EO)ₙ(PO)ₘH (I)
in which R¹ is an alkyl group containing 6 to 22 carbon atoms, EO stands for CH₂CH₂O, PO stands for CHCH₃CH₂O and/or CH₂CHCH₃O, n is a whole or broken number of 2 to 7 and m is a whole or broken number of 1.5 to 3,
the molar ratio of propylene oxide to ethylene oxide being from 10:90 to 50:50,
as surfactants in water-dilutable concentrates of active substances, **characterized in that** the concentrates contain pesticides or agrochemicals.

2. The use claimed in claim 1, **characterized in that** the molar ratio of propylene oxide to ethylene oxide is from 25:75 to 40:60.

3. The use claimed in claim 1, **characterized in that** n is a whole or broken number of 3 to 5.

4. The use claimed in claim 1, **characterized in that** m is a whole or broken number of 2 to 2.5.

5. The use claimed in claim 1, **characterized in that** R¹ is derived from a fatty alcohol mixture containing at least 30% by weight of C₁₄₋₁₈ fatty alcohols and at most 70% by weight of C₆₋₁₂ fatty alcohols.

## Revendications

1. Utilisation de polymères statistiques d'alcools gras avec de l'oxyde d'éthylène et de l'oxyde de propylène de formule (I)
R¹O(EO)ₙ(PO)ₘH (I)
dans laquelle R¹ représente un reste alkyle ayant de 6 à 22 atomes de carbone,
EO représente CH₂CH₂O
PO représente CHCH₃CH₂O et/ou CH₂CHCH₃O
n représente un nombre entier ou fractionnaire allant de 2 à 7 et
m représente un nombre entier ou fractionnaire allant de 1,5 à 3,
dont le rapport molaire de l'oxyde de propylène à l'oxyde d'éthylène se situe dans la plage de 10 :90 à 50 :50,
en tant qu'agent tensioactif dans des concentrés de principes actifs qu'on peut diluer avec de l'eau,
**caractérisée en ce que**
les concentrés de principe actif renferment des pesticides ou des produits agrochimiques.

2. Utilisation selon la revendication 1,
**caractérisée en ce que**
le rapport molaire de l'oxyde de propylène à l'oxyde d'éthylène se situe dans la plage de 25 : 75 à 40 : 60.

3. Utilisation selon la revendication 1,
**caractérisée en ce que**
n est un nombre entier ou fractionnaire situé dans la plage de 3 à 5.

4. Utilisation selon la revendication 1,
**caractérisée en ce que**
m est un nombre entier ou fractionnaire situé dans la plage de 2 à 2,5.

5. Utilisation selon la revendication 1,
**caractérisée en ce que**
R¹ est dérivé d'un mélange d'alcools gras qui renferme au moins jusqu'à 30 % en poids d'alcools gras ayant de 14 à 18 atomes de carbone et au maximum 70 % en poids d'alcools gras ayant de 6 à 12 atomes de carbone.
